# EUROPÄISCHE PATENTSCHRIFT

(11) **EP 1 020 436 B1**
(45) Veröffentlichungstag und Bekanntmachung des Hinweises auf die Patenterteilung: **26.03.2003**
(21) Anmeldenummer: 99125848.4
(22) Anmeldetag: 24.12.1999
(51) Int. Cl.: C07C 303/32, C07C 303/22, C07C 309/42

(54) **Verfahren zur Herstellung von Amidosäurephenylestern**
Process for the preparation of amidocarboxylic acid phenyl esters
Procédé pour la préparation d'esters phényliques d'acides amidocarboxyliques

(30) Priorität: 13.01.1999 DE 19900939
(43) Veröffentlichungstag der Anmeldung: 19.07.2000
(73) Patentinhaber: Clariant GmbH, 65929 Frankfurt am Main (DE)
(72) Erfinder: Seebach, Michael, Dr., 65795 Hattersheim (DE); Naumann, Peter, Dipl.-Ing., 65232 Taunusstein (DE); Bäumler, Uwe, Dr., 60386 Frankfurt (DE)

(56) Entgegenhaltungen:
- WO-A-94/28106
- US-A- 5 523 434

## Beschreibung

Die Erfindung betrifft die Synthese von Amidosäurephenylestern durch Umsetzung von Amidocarbonsäuren mit anorganischen Säurehalogeniden und in einem weiteren Reaktionsschritt mit einem Phenolderivat.

Amidosäurephenylester werden als Bleichaktivatoren in Wasch- und Reinigungsmitteln eingesetzt. Sie ermöglichen eine bleichende Wirkung bereits bei Temperaturen unter 60°C, indem sie mit einer Quelle für Wasserstoffperoxid - meist Perborate oder Percarbonate - unter Freisetzung einer organischen Peroxysäure reagieren.

In der Patentliteratur werden unterschiedliche Syntheseverfahren dieser Bleichaktivatoren beschrieben.
So beschreibt US-A-5 523 434 die Herstellung von Amidosäurephenylestern aus Amidocarbonsäuren und Phenolsulfonaten durch ein zweistufiges Verfahren: Im ersten Schritt erfolgt die Synthese eines Amidocarbonsäurechlorides durch Umsetzung der Amidocarbonsäure mit anorganischen Säurechloriden und in einem zweiten Schritt die Reaktion des Amidocarbonsäurechlorids mit einem Phenolsulfonat in einem Wasser/Diethylethergemisch. Problematisch für die großtechnische Anwendbarkeit dieses Verfahrens ist der Einsatz von Diethylether als Lösungsmittel. Nachteilig sind weiterhin geringe Ausbeuten und die Verwendung großer Überschüsse an anorganischem Säurechlorid bei der Synthese des Amidocarbonsäuredichlorids.

In US-A-5 466 840 wird ebenfalls ein mehrstufiges Syntheseverfahren von Amidosäurephenylestersulfonaten beschrieben. Darin wird das Alkalisalz einer 4-Hydroxybenzolsulfonsäure mit einem C₂-C₄-Carbonsäureanhydrid zum Alkalisalz einer 4-Acyloxybenzolsulfonsäure umgesetzt. Diese wird im zweiten Schritt durch Zugabe von 1-Oxyalkanoylaminocarbonsäure in Gegenwart eines Umesterungs-Katalysators bei 150 bis 250°C innerhalb von 0,5 bis 10 Stunden zum Amidosäurephenylestersulfonat überführt. Die Bildung von Nebenprodukten, Ausbeuteverluste und aufwendige Reinigungsverfahren der Produkte verteuern die Herstellung dieser als Bleichaktivatoren in Wasch- und Reinigungsmitteln einzusetzenden Stoffklasse.

In dem Verfahren gemäß WO 96/39378 werden Amidocarbonsäure und ein Phenolderivat in Sulfolan vorgelegt, ein Carbonsäureanhydrid, beispielsweise Acetanhydrid, zugetropft und durch Erhitzen auf ca. 170°C die Umsetzung zu Amidosäurephenylestersulfonaten je nach Ausgangsverbindung innerhalb von 0,5 bis 10 Stunden erreicht.

Unbefriedigend ist der sehr hohe Energieaufwand während der Reaktionsführung, verminderte Ausbeuten, stark verunreinigte Produkte sowie die sehr aufwendige und kostenintensive Abtrennung des hoch siedenden Lösungsmittels und ein sehr aufwendiges Recycling von Abgasen, Nebenprodukten und Lösungsmitteln. Es bestand daher die Aufgabe, eine verbesserte Verfahrensweise zur Herstellung von Amidosäurephenylestersulfonaten zu finden.

Es wurde gefunden, daß durch langsames Zutropfen eines Säurehalogenids bei 20 bis 130°C zu einer lösungsmittelfreien Schmelze von Amidocarbonsäure und einer weiteren Umsetzung des entgasten Zwischenproduktes Amidocarbonsäurehalogenid mit wasserfreiem Phenolsulfonat oder einem Phenolderivat, suspendiert in einem polaren, aprotischen Lösungsmittel bei 20 bis 130°C, Amidosäurephenylestersulfonate in sehr reiner Form und in hohen Ausbeuten erhalten werden.
Vorteilhaft ist ein wesentlich geringerer Energieaufwand als bei den gängigen Verfahren, einfache Entfernung des Lösungsmittels durch Fällung des Produktes, lösungsmittelfreie und damit leicht wiederverwertbare Abgase, sowie eine Reduzierung von Nebenprodukten, insbesondere von Salzen.

Gegenstand der Erfindung ist ein Verfahren zur Synthese von Amidosäurephenylestern der Formel I 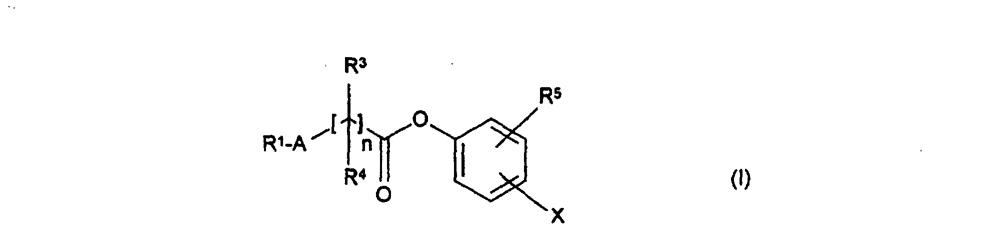 wobei
A eine Gruppe der Formel -CONR²- oder -NR²CO-,
R¹ C₁-C₂₆-Alkyl, C₂-C₂₆-Alkenyl, C₂-C₂₆-Alkinyl oder C₃-C₈-Cycloalkyl oder eine Aryloder Alkylarylgruppe mit jeweils 6 bis 14 C-Atomen,
R² Wasserstoff oder C₁-C₂₆-Alkyl, C₂-C₂₆-Alkenyl, C₂-C₂₆-Alkinyl oder C₃-C₈-Cycloalkyl oder eine Aryl- oder Alkylarylgruppe mit jeweils 6 bis 14 C-Atomen,
R³ und R⁴ die gleich oder verschieden sein können, jeweils Wasserstoff oder C₁-C₁₀-Alkyl, C₂-C₁₀-Alkenyl, C₂-C₁₀-Alkinyl oder C₃-C₈-Cycloalkyl,
R⁵ Wasserstoff, Halogen oder C₁-C₆-Alkyl, C₂-C₆-Alkenyl, C₂-C₆-Alkinyl, C₃-C₈-Cycloalkyl oder C₁-C₆-Alkoxy bedeuten, n für eine Zahl von 1 bis 10 steht,
X eine Gruppe der Formeln SO₃M, OSO₃M, (CH₂)ₘSO₃M, (CH₂)ₘ-OSO₃M, CO₂M und N(R⁶)₃Y bedeutet,
wobei M Wasserstoff oder ein Alkaliion,
R⁶ eine Alkylgruppe mit 1 bis 6 C-Atomen oder eine Cycloalkylgruppe mit 3 bis 8 C-Atomen,
Y ein Halogenatom und
m 1 oder 2 bedeutet.

Bevorzugt ist die Herstellung der Verbindungen der Formel (I) wobei gleichzeitig A eine Gruppe der Formel -CONR²-, R¹ C₆-C₁₀-Alkyl, R², R³, R⁴ und R⁵ Wasserstoff, n = 5 und X -SO₃M bedeuten.

Dieses Verfahren besteht darin, daß man ein anorganisches Säurehalogenid zu einer Schmelze der Verbindung der Formel II 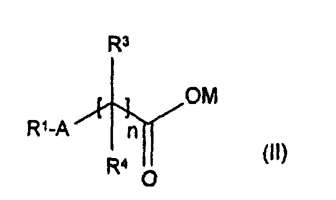 gibt, das entstandene Amidocarbonsäurehalogenid im Vakuum von verbleibenden Abgasen befreit, und dieses Amidocarbonsäurehalogenid in einem zweiten Reaktionsschritt mit einem Phenolderivat der Formel III, 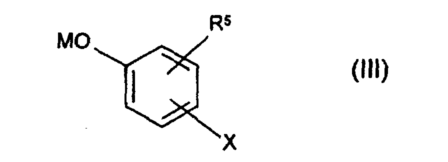 suspendiert in einem geeigneten organischen Lösungsmittel, umsetzt.

In der ersten Reaktionsstufe wird die Verbindung der Formel I aufgeschmolzen und zu der Schmelze wird langsam unter Rühren ein anorganisches Säurehalogenid zugetropft.

Als anorganisches Säurehalogenid kann beispielsweise PCl₃, PCl₅, POCl₃, COCl₂, bevorzugt SOCl₂ eingesetzt werden. Anstelle dieser Chloride können auch die analogen Bromide eingesetzt werden. Die Menge an Säurehalogenid beträgt 0,5 bis 2, bevorzugt 0,7 bis 1,5, insbesondere 0,9 bis 1,4 Moläquivalente bezogen auf die Amidocarbonsäure. Die Temperatur, bei der die Reaktion durchgeführt wird ist abhängig vom Schmelzpunkt der Amidocarbonsäure und liegt im allgemeinen bei 20 bis 120°C, bevorzugt bei 50 bis 110°C, besonders bevorzugt bei 70 bis 100°C. Die Reaktion läuft für den ersten Verfahrensschritt in einem Zeitraum von 10 Minuten bis 5 Stunden, bevorzugt 30 Minuten bis 3 Stunden ab, dem sich eine Nachrührzeit von 10 Minuten bis 3 Stunden, bevorzugt 30 Minuten bis 2 Stunden, anschließt. Nach Beendigung der Reaktion wird Vakuum angelegt, um die entstandenen Abgase, insbesondere SO₂, HCI und Reste anorganischen Säurehalogenids zu entfernen.

Im zweiten Reaktionsschritt wird eine Schmelze des im ersten Verfahrensschritt erhaltenen Säurehalogenids vorgelegt und zu dieser Schmelze wird unter Inertgas langsam eine Suspension der Verbindung III in einem geeigneten Lösungsmittel zugegeben.

Als Lösungsmittel kommen beispielsweise infrage Xylol, Benzol, Monoglyme, Diglyme, Diisopropylether, Tetrahydrofuran, Dioxan, Isobutylmethylketon, Aceton, Diethylketon, Acetonitril, Fettsäurealkylester, bevorzugt Essigsäure-C₂-C₄-alkylester. Beispiele für diese Lösungsmittel sind Essigsäure-ethylester, Essigsäure-npropylester, Essigsäure-i-propylester, Essigsäure-n-butylester, Essigsäure-ibutylester, Essigsäure-t-butylester oder deren Gemische. Bevorzugt ist n-Butylacetat, da dieses Lösungsmittel eine sehr gute Löslichkeit der Ausgangsprodukte und ein gutes Kristallisationsverhalten des Endprodukts aufweist. Das molare Verhältnis der Verbindungen der Formeln II und III beträgt 1:0,7 bis 1,5, bevorzugt 1:0,8 bis 1,3.
Die Reaktionszeit im zweiten Verfahrensschritt beträgt ungefähr 1 Minute bis 2 Stunden, bevorzugt 15 bis 90 Minuten. Daran schließt sich eine Nachrührzeit von 1 Minute bis zu 2 Stunden an. Die Reaktionstemperatur in der zweiten Stufe liegt im allgemeinen bei 15 bis 110°C.

Die Reaktionsmischung wird nach Verdünnen mit Wasser durch Zugabe einer Base, bevorzugt Natronlauge, Kalilauge, Natriumcarbonat oder Kaliumcarbonat auf einen pH-Wert von pH 4 bis pH 11, bevorzugt pH 7 bis pH 10 eingestellt. Die dabei erhaltenen Endprodukte können durch Filtration, Abnutschen, Abdekantieren oder durch Zentrifugation aus dieser Mutterlauge abgetrennt werden. Zur Reinigung kann das feuchte Produkt mit Wasser, Alkoholen, aromatischen Lösungsmitteln, Alkanen, Ketonen oder Estern, sowie Gemischen aus diesen, vorzugsweise mit Wasser, Alkoholen oder Estern oder deren Gemischen ausgerührt oder umkristallisiert werden.

Nach dem erfindungsgemäßen Syntheseverfahren wird die Zielverbindung in Ausbeuten von über 80 % und hoher Reinheit (Gehalt an Amidosäurephenylester über 90 %) erhalten.

Nachfolgende Beispiele sollen die Erfindung näher erläutern ohne sie darauf einzuschränken.

### Beispiel 1

135,7 g (0,5 mol) n-Nonanoylamidohexansäure wurden vorgelegt und bei einer Temperatur von 85°C bis 90°C aufgeschmolzen. 59,5 g (0,5 mol) Thionylchlorid wurden innerhalb von 2 Stunden der Schmelze zudosiert. Das Reaktionsgemisch wurde 1 Stunde bei Raumtemperatur nachgerührt und im Wasserstrahlvakuum im Zeitraum von 1,5 Stunden entgast. Das entstandene n-Nonanoylamidohexansäurechlorid wurde auf 85°C bis 90°C erhitzt und 100,1 g (0,5 mol) wasserfreies p-Phenolsulfonsäure-Natriumsalz, suspendiert in 300 ml n-Butylacetat, unter Stickstoff-Atmosphäre innerhalb einer Stunde zudosiert. Das Reaktionsgemisch wurde für 1 Stunde bei 85 bis 90°C nachgerührt.
Nach dem Abkühlen auf ca. 30°C wurden 900 ml Wasser unter starkem Rühren zugegeben. Durch Zutropfen von 128 g Natronlauge (32 %ig) bei einer Temperatur von 35 bis 40°C wurde ein pH-Wert von 8,0 eingestellt. Das ausgefallene Produkt wurde auf 20°C abgekühlt und abgesaugt, einmal mit Wasser gewaschen und über Nacht bei 60°C im Vakuumtrockenschrank getrocknet.
Es wurden 87 % n-Nonanoylamidocaproyl-oxy-benzolsulfonsäure-Natriumsalz als weißer Feststoff erhalten.

### Beispiel 2

135,7 g (0,5 mol) n-Nonanoylamidohexansäure wurden vorgelegt und bei einer Temperatur von 85°C bis 90°C aufgeschmolzen. 59,5 g (0,5 mol) Thionylchlorid wurden innerhalb von 2 Stunden der Schmelze zudosiert. Das Reaktionsgemisch wurde 1 Stunde bei Raumtemperatur nachgerührt und im Wasserstrahlvakuum im Zeitraum von 1,5 Stunden entgast. Das entstandene n-Nonanoylamidohexansäurechlorid wurde auf 90°C erhitzt und 100,1 g (0,5 mol) wasserfreies p-Phenolsulfonsäure-Natriumsalz, suspendiert in 300 ml n-Butylacetat, unter Stickstoff-Atmosphäre innerhalb von 5 Minuten zudosiert. Das Reaktionsgemisch wurde 30 Minuten bei 90°C nachgerührt.
Nach dem Abkühlen auf ca. 30°C wurden 900 ml Wasser unter starkem Rühren zugegeben. Durch Zutropfen von 123 g Natronlauge (32 %ig) bei einer Temperatur von 35 bis 40°C wurde ein pH-Wert von 8,0 eingestellt. Das ausgefallene Produkt wurde auf 20°C abgekühlt und abgesaugt, einmal mit Wasser gewaschen und über Nacht bei 60°C im Vakuumtrockenschrank getrocknet.
Es wurden 86 % n-Nonanoylamidocaproyl-oxy-benzolsulfonsäure-Natriumsalz als weißer Feststoff erhalten.

## Patentansprüche

1. Verfahren zur Herstellung von Amidosäurephenylestern der Formel I 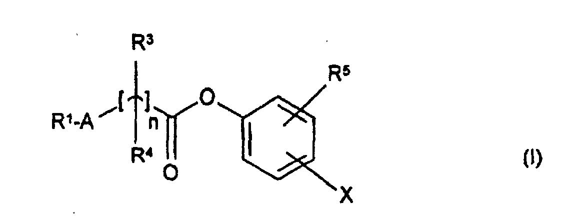 wobei
A eine Gruppe der Formel -CONR²- oder -NR²CO-,
R¹ C₁-C₂₆-Alkyl, C₂-C₂₆-Alkenyl, C₂-C₂₆-Alkinyl oder C₃-C₈-Cycloalkyl oder eine Aryloder Alkylarylgruppe mit jeweils 6 bis 14 C-Atomen,
R² Wasserstoff oder C₁-C₂₆-Alkyl, C₂-C₂₆-Alkenyl, C₂-C₂₆-Alkinyl oder C₃-C₈-Cycloalkyl oder eine Aryl- oder Alkylarylgruppe mit jeweils 6 bis 14 C-Atomen,
R³ und R⁴ die gleich oder verschieden sein können, jeweils Wasserstoff oder C₁-C₁₀-Alkyl, C₂-C₁₀-Alkenyl, C₂-C₁₀-Alkinyl oder C₃-C₈-Cycloalkyl.
R⁵ Wasserstoff, Halogen oder C₁-C₆-Alkyl, C₂-C₆-Alkenyl, C₂-C₆-Alkinyl, C₃-C₈-Cycloalkyl oder C₁-C₆-Alkoxy bedeuten, n für eine Zahl von 1 bis 10 steht,
X eine Gruppe der Formeln SO₃M, OSO₃M, (CH₂)ₘSO₃M, (CH₂)ₘ-OSO₃M, CO₂M und N(R⁶)₃Y bedeutet,
wobei M Wasserstoff oder ein Alkaliion,
R⁶ eine Alkylgruppe mit 1 bis 6 C-Atomen oder eine Cycloalkylgruppe mit 3 bis 8 C-Atomen,
Y ein Halogenatom und
m 1 oder 2 bedeutet,
**dadurch gekennzeichnet, daß** man ein anorganisches Säurehalogenid zu einer Schmelze der Verbindung der Formel II 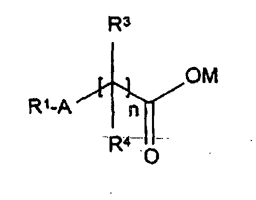 gibt, das entstandene Amidocarbonsäurehalogenid im Vakuum von verbleibenden Abgasen befreit, und dieses Amidocarbonsäurehalogenid in einem zweiten Reaktionsschritt mit einem Phenolderivat der Formel III, 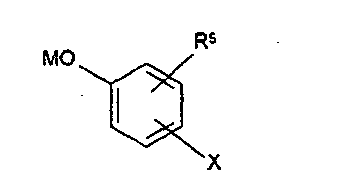 suspendiert in einem geeigneten organischen Lösungsmittel, umsetzt.

2. Verfahren nach Anspruch 1, **dadurch gekennzeichnet, daß** man mit einem molaren Verhältnis der Verbindungen II und III von 1:0,7 bis 1,5 arbeitet.

3. Verfahren nach Anspruch 1, **dadurch gekennzeichnet, daß** man mit einem molaren Verhältnis der Verbindungen II und III von 1:0,8 bis 1,3 arbeitet.

4. Verfahren nach Anspruch 1, **dadurch gekennzeichnet, daß** man mit Toluol als Lösungsmittel arbeitet.

5. Verfahren nach Anspruch 1, **dadurch gekennzeichnet, daß** man mit Acetonitril als Lösungsmittel arbeitet.

6. Verfahren nach Anspruch 1, **dadurch gekennzeichnet, daß** man mit Essigsäure-C₁-C₄-alkylester als Lösungsmittel arbeitet.

7. Verfahren nach Anspruch 1, **dadurch gekennzeichnet, daß** man mit n-Butylacetat als Lösungsmittel arbeitet.

8. Verfahren nach Anspruch 1, **dadurch gekennzeichnet, daß** die Menge an Säurehalogenid 0,5 bis 2 Moläquivalente, bezogen auf Amidocarbonsäure II, beträgt.

9. Verfahren nach Anspruch 1, **dadurch gekennzeichnet, daß** die Menge an Säurehalogenid 0,7 bis 1,5 Moläquivalente, bezogen auf Amidocarbonsäure II, beträgt.

10. Verfahren nach Anspruch 1, **dadurch gekennzeichnet, daß** die Menge an Säurehalogenid 0,9 bis 1,4 Moläquivalente, bezogen auf Amidocarbonsäure II, beträgt.

11. Verfahren nach Anspruch 1, **dadurch gekennzeichnet, daß** man bei 25 bis 120°C arbeitet.

## Revendications

1. Procédé pour la préparation d'esters phényliques d'acides amidocarboxyliques de formule I 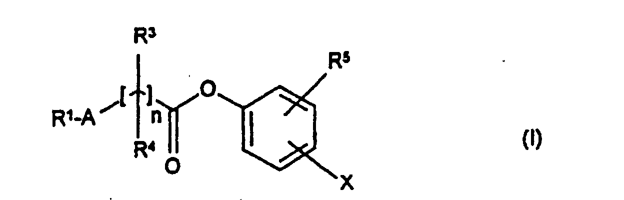 dans laquelle
A représente un groupe de formule -CONR²- ou -NR²CO-,
R¹ représente un groupe alkyle en C₁ à C₂₆, alcényle en C₂ à C₂₆, alcynyle en C₂ à C₂₆ ou cycloalkyle en C₃ à C₈, ou un groupe aryle ou alkylaryle avec respectivement 6 à 14 atomes de carbone,
R² représente un atome d'hydrogène ou un groupe alkyle en C₁ à C₂₆, alcényle en C₂ à C₂₆, alcynyle en C₂ à C₂₆ ou cycloalkyle en C₃ à C₈ ou un groupe aryle ou alkylaryle avec respectivement 6 à 14 atomes de carbone,
R³ et R⁴, qui peuvent être identiques ou différents, représentent respectivement un atome d'hydrogène ou un groupe alkyle en C₁ à C₁₀, alcényle en C₂ à C₁₀, alcynyle en C₂ à C₁₀ ou cycloalkyle en C₃ à C₈,
R⁵ représente un atome d'hydrogène, d'halogène ou un groupe alkyle en C₁ à C₆, alcényle en C₂ à C₆, alcynyle en C₂ à C₆, cycloalkyle en C₃ à C₈ ou alcoxy en C₁ à C₆, n représente un nombre de 1 à 10,
X représente un groupe des formules SO₃M, OSO₃M, (CH₂)ₘSO₃M, (CH₂)ₘ-OSO₃M, CO₂M et N(R⁶)₃Y, dans lesquelles M représente un atome d'hydrogène ou un ion alcalin,
R⁶ représente un groupe alkyle avec de 1 à 6 atomes de carbone ou un groupe cycloalkyle avec de 3 à 8 atomes de carbone,
Y représente un atome d'halogène, et
m représente 1 ou 2,
**caractérisé en ce qu'**on ajoute un halogénure d'acide inorganique à une matière fondue du composé de formule II 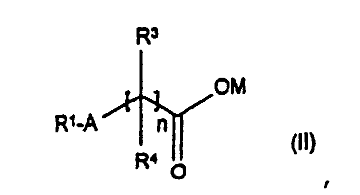 on libère l'halogénure d'acide amidocarboxylique produit sous vide des gaz d'échappement restants, et on met à réagir cet halogénure d'acide amidocarboxylique dans une deuxième étape de réaction avec un dérivé de phénol de formule III, 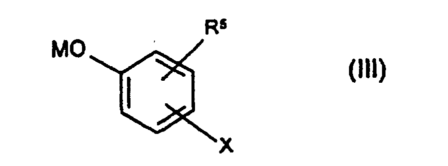 mis en suspension dans un solvant organique approprié.

2. Procédé selon la revendication 1, **caractérisé en ce qu'**on travaille avec un rapport molaire des composés II et III de 1:0,7 jusqu'à 1,5.

3. Procédé selon la revendication 1, **caractérisé en ce qu'**on travaille avec un rapport molaire des composés II et III de 1:0,8 jusqu'à 1,3.

4. Procédé selon la revendication 1, **caractérisé en ce qu'**on travaille avec du toluène comme solvant.

5. Procédé selon la revendication 1, **caractérisé en ce qu'**on travaille avec l'acétonitrile comme solvant.

6. Procédé selon la revendication 1, **caractérisé en ce qu'**on travaille avec un ester alkylique en C₁ à C₄ d'acide acétique comme solvant.

7. Procédé selon la revendication 1, **caractérisé en ce qu'**on travaille avec l'acétate de n-butyle comme solvant.

8. Procédé selon la revendication 1, **caractérisé en ce que** la quantité d'halogénure d'acide s'élève à 0,5 jusqu'à 2 équivalents molaires, par rapport à l'acide amidocarboxylique II.

9. Procédé selon la revendication 1, **caractérisé en ce que** la quantité d'halogénure d'acide s'élève à 0,7 jusqu'à 1,5 équivalent molaire, par rapport à l'acide amidocarboxylique II.

10. Procédé selon la revendication 1, **caractérisé en ce que** la quantité d'halogénure d'acide s'élève à 0,9 jusqu'à 1,4 équivalent molaire, par rapport à l'acide amidocarboxylique II.

11. Procédé selon la revendication 1, **caractérisé en ce qu'**on travaille de 25 jusqu'à 120 °C.

## Claims

1. A process for preparing amido acid phenyl esters of the formula I 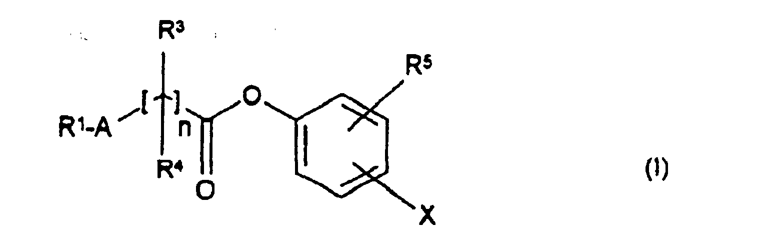 where
A is a group of the formula -CONR²- or -NR²CO-,
R¹ is C₁-C₂₆-alkyl, C₂-C₂₆-alkenyl, C₂-C₂₆-alkynyl or C₃-C₈-cycloalkyl or an aryl or alkylaryl group each having from 6 to 14 carbon atoms,
R² is hydrogen or C₁-C₂₆-alkyl, C₂-C₂₆-alkenyl, C₂-C₂₆-alkynyl or C₃-C₈-cycloalkyl or an aryl or alkylaryl group each having from 6 to 14 carbon atoms,
R³ and R⁴, which can be identical or different, are each hydrogen or C₁-C₁₀-alkyl, C₂-C₁₀-alkenyl, C₂-C₁₀-alkynyl or C₃-C₈-cycloalkyl,
R⁵ is hydrogen, halogen or C₁-C₆-alkyl, C₂-C₆-alkenyl, C₂-C₆-alkynyl, C₃-C₈-cycloalkyl or C₁-C₆-alkoxy, n is a number from 1 to 10,
X is a group of the formulae SO₃M, OSO₃M, (CH₂)ₘSO₃M, (CH₂)ₘOSO₃M, CO₂M and N(R⁶)₃Y,
where M is hydrogen or an alkali metal ion,
R⁶ is an alkyl group having from 1 to 6 carbon atoms or a cycloalkyl group having from 3 to 8 carbon atoms,
Y is a halogen atom and
m is 1 or 2,
which comprises adding an inorganic acid halide to a melt of the compound of the formula II 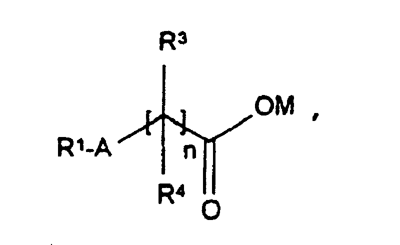 freeing the resulting amidocarboxylic acid halide under reduced pressure from waste gases which remain, and reacting this amidocarboxylic acid halide in a second reaction step with a phenol derivative of the formula III 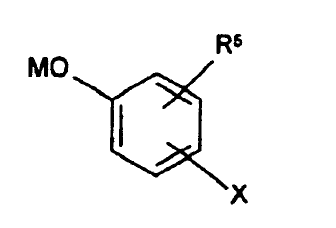 suspended in a suitable organic solvent.

2. The process as claimed in claim 1, wherein the molar ratio of the compounds II and III is from 1:0.7 to 1.5.

3. The process as claimed in claim 1, wherein the molar ratio of the compounds II and III is from 1:0.8 to 1.3.

4. The process as claimed in claim 1, wherein toluene is used as solvent.

5. The process as claimed in claim 1, wherein acetonitrile is used as solvent.

6. The process as claimed in claim 1, wherein a C₁-C₄-alkyl acetate is used as solvent.

7. The process as claimed in claim 1, wherein n-butyl acetate is used as solvent.

8. The process as claimed in claim 1, wherein the amount of acid halide is from 0.5 to 2 mol equivalents, based on amidocarboxylic acid II.

9. The process as claimed in claim 1, wherein the amount of acid halide is from 0.7 to 1.5 mol equivalents, based on amidocarboxylic acid II.

10. The process as claimed in claim 1, wherein the amount of acid halide is from 0.9 to 1.4 mol equivalents, based on amidocarboxylic acid II.

11. The process as claimed in claim 1, which is carried out at from 25 to 120°C.
